# EUROPEAN PATENT SPECIFICATION

(11) **EP 1 141 403 B1**
(45) Date of publication and mention of the grant of the patent: **09.10.2002**
(21) Application number: 99964776.1
(22) Date of filing: 28.12.1999
(51) Int. Cl.: C12Q 1/68

(54) **DNA OLIGONUCLEOTIDE WHICH IS SPECIFIC FOR MELOIDOGYNE SPECIES, DNA VECTOR AND HOST CELL CONTAINING THE OLIGONUCLEOTIDE, USE AND KIT**
DNS OLIGONUKLEOTID SPEZIFISCH FÜR MELOIDOGYNE GATTUNGEN, DNS VEKTOR UND WIRTSORGANISMUS DIE ES BEINHALTEN, VERWENDUNGEN UND ZUSAMENSETZUNG
OLIGONUCLEOTIDE D'ADN, SPECIFIQUE DE L'ESPECE MELOIDOGYNE, VECTEUR D'ADN ET CELLULE HOTE CONTENANT CET OLIGONUCLEOTIDE, UTILISATION ET TROUSSE

(30) Priority: 30.12.1998 NL 1010917
(43) Date of publication of application: 10.10.2001
(73) Proprietor: Plant Research International B.V., 6708 PB Wageningen (NL)
(72) Inventor: ZIJLSTRA, Carolien, NL-6721 ZJ Bennekom (NL)
(74) Representative: Van kan, Johan Joseph Hubert, Ir.
(86) International application number: NL9900812
(87) International publication number: WO00040754

(56) References cited:
- PETERSEN, D. ET AL: "Species-specific probes for the detection of Meloidogyne chitwoodi and M. fallax." JOURNAL OF NEMATOLOGY, (DEC., 1997) VOL. 29, NO. 4, PP. 599. MEETING INFO.: 36TH ANNUAL MEETING OF THE SOCIETY OF NEMATOLOGISTS TUCSON, ARIZONA, USA JULY 19-23, 1997 SOCIETY OF NEMATOLOGISTS., XP002112997
- ZIJLSTRA, CAROLIEN (1): "A fast PCR assay to identify Meloidogyne hapla, M. chitwoodi, and M. fallax, and to sensitively differentiate them from each other and from M. incognita in mixtures." FUNDAMENTAL AND APPLIED NEMATOLOGY, (1997) VOL. 20, NO. 5, PP. 505-511., XP002112998
- BLOK, VIVIAN C. (1) ET AL: "Genetic variation in tropical Meloidogyne spp. as shown by RAPDs." FUNDAMENTAL AND APPLIED NEMATOLOGY, (1997) VOL. 20, NO. 2, PP. 127-133., XP002112999
- WILLIAMSON, V. M. ET AL: "A PCR assay to identify and distinguish single juveniles of Meloidogyne hapla and M. chitwoodi." JOURNAL OF NEMATOLOGY, (1997) VOL. 29, NO. 1, PP. 9-15., XP002113000 cited in the application
- PETERSEN, DANIEL J. (1) ET AL: "Rapid identification of Meloidogyne chitwoodi, M. hapla, and M. fallax using PCR primers to amplify their ribosomal intergenic spacer." FUNDAMENTAL AND APPLIED NEMATOLOGY, (1996) VOL. 19, NO. 6, PP. 601-605., XP002113001
- CENIS, J. L.: "Identification of four major Meloidogyne spp. by random amplified polymorphic DNA (RAPD-PCR." PHYTOPATHOLOGY, (1993) VOL. 83, NO. 1, PP. 76-78., XP002113003
- POWERS, T. O. ET AL: "A polymerase chain reaction method for identification of five major Meloidogyne species." JOURNAL OF NEMATOLOGY, (1993) VOL. 25, NO. 1, PP. 1-6., XP002113004
- CASTAGNONE-SERENO P ET AL: "Genetic polymorphism between and within Meloidogyne species detected with RAPD markers." GENOME, (1994 DEC) 37 (6) 904-9., XP002113002
- CASTAGNONE-SERENO, P. (1) ET AL: "Specific diagnosis of two root-knot nematodes, Meloidogyne chitwoodi and M. fallax, with satellite DNA probes." PHYTOPATHOLOGY, (MAY, 1999) VOL. 89, NO. 5, PP. 380-384., XP002137602

## Description

The present invention relates to a DNA oligonucleotide which is specific for Meloidogyne species.

Such an oligonucleotide is known from Williamson, V.M. Caswell-Chen, E.P., Westerdahl, B.B., Wu, F.F., en Garyl, G. (1997), Journal of Nematology 29, 9-15. In Williamson e.a. (1997), the search for, and the preparation of DNA oligonucleotides which are specific for Meloidogyne species is described. Only for two Meloidogyne species, i.e. M. hapla and M. chitwoodi, are DNA oligonucleotides, two in each case, found and described which can be used for a species-specific determination. While searching for oligonucleotides with a specificity for other species, variable and/or non-species specific results were obtained. Incidentally, research carried out by applicant shows that the DNA oligonucleotides which are mentioned for M. chitwoodi are not species-specific, as they undesirably also react with DNA from M. fallax.

Root-knot nematodes, which are nematodes from the genus Meloidogyne, are harmful parasites causing damage to crops such as potatoes, beets, black salsifies (scorzoneras) and carrots, and thus lead to considerable crop losses. The damage caused is so serious that, on a European level, M. fallax and M. chitwoodi were given a quarantine status in 1998. It has been observed that the spread and the damage of different Meloidogyne species has increased in recent years. Therefore, a demand for more truly species-specific oligonucleotides, which can be used for a species-specific detection and identification of different Meloidogyne species, exists in the art.

The present invention provides a DNA oligonucleotide which is specific for Meloidogyne species, which is characterised in that the DNA oligonucleotide has the following sequence: (a) a DNA sequence chosen from the group consisting of SEQ ID No. 1 to SEQ ID No. 20, or (b) a DNA sequence which under conditions of stringency hybridises with a sequence according to (a) and which is specific for Meloidogyne species.

From DATABASE GENESEQ 'Online!, Derwent, AC:T51964, an RNA oligonucleotide, representing a preferred human ICAM-1 hammerhead ribozyme target sequence, is known.

Using the DNA oligonucleotides according to the present invention, it is possible to determine the presence of various Meloidogyne species. This is important for, among others, the crop grower, who wants to know whether there are any root-knot nematodes in his parcels, and, if so, also wants to identify the species involved. For this process specific, reliable and sensitive detection tests are needed. Obviously, this applies particularly for species with a quarantine status, as the presence of such species, i.e. in seed material for export, has to be checked routinely. Furthermore, an effective resistance and/or virulence analysis will only be possible on the basis of a specific detection, identification and determination of the species composition in a sample.

The present invention not only relates to DNA oligonucleotides which are described in the added sequence listing, but also to such sequences which hybridise thereto in certain conditions of stringency. Generally, the conditions of stringency as meant within the framework of the present invention comprise a hybridisation in 6 x SSC (1 x SSC = 0.15 M NaCl, 0.015 M sodium citrate, pH 7.0), 0.1% SDS at a temperature of 40 °C. Oligonucleotides hybridising in 6 x SSC, 0.1% SDS at 55 °C are more homologous to the DNA oligonucleotides described in the sequence listing, and oligonucleotides hybridising in 2 x SSC at 65 °C are particularly preferable, as they will be the most homologous.

Oligonucleotides consisting of a combination of the oligonucleotides with SEQ ID No. 5 and No. 2, SEQ ID No. 6 and No. 7 and SEQ ID No. 14 and No. 11 are preferable, as they can be used for a simultaneous species-specific detection of three Meloidogyne species, i.e. M. fallax, M. hapla and M. chitwoodi. Processes for detecting and identifying these and other Meloidogyne species, using DNA oligonucleotides according to the present invention, are described hereinafter and in the Examples.

For specifically detecting and identifying M. fallax, the oligonucleotide with a sequence chosen from SEQ ID No. 1 and SEQ ID No. 2 is particularly suitable. The following oligonucleotides are preferred for specifically detecting and identifying other Meloidogyne species: the oligonucleotide with a sequence chosen from SEQ ID No. 6 and SEQ ID No. 7 for M. hapla, the oligonucleotide with a sequence chosen from SEQ ID No. 10 and SEQ ID No. 11 for M. chitwoodi, the oligonucleotide with a sequence chosen from SEQ ID No. 15 and SEQ ID No. 16 for M. arenaria, the oligonucleotide with a sequence chosen from SEQ ID No. 17 and SEQ ID No. 18 for M. incognita, and the oligonucleotide with a sequence chosen from SEQ ID No. 19 and SEQ ID No. 20 for M. javanica.

According to the present invention, it is possible to increase the sensitivity of the detection of three of the aforementioned Meloidogyne species considerably, by using a follow-up reaction which is described hereinafter and in the Examples. For increasing the sensitivity of the detection of M. fallax, a DNA oligonucleotide with a sequence chosen from SEQ ID No. 3 and SEQ ID No. 4 is preferred. For increasing the sensitivity of the detection of M. hapla, a DNA oligonucleotide with a sequence chosen from SEQ ID No. 8 and SEQ ID No. 9 is preferred, and finally, a DNA oligonucleotide with a sequence chosen from SEQ ID No. 12 and SEQ ID No. 13 is preferred for increasing the sensitivity of the detection of M. chitwoodi.

The invention also relates to a DNA vector comprising a DNA oligonucleotide according to the invention, as well as to a prokaryotic or eukaryotic host cell comprising such vector. In the context of the present invention "DNA vector" is meant to be a natural DNA species or an artificial DNA construct, suitable to express or amplify an incorporated DNA sequence (for instance a DNA oligonucleotide according to the invention) in a suitable host cell. Examples of DNA vectors are plasmids and viruses, such as phages. An example of a host cell is a bacterium, for instance E. coli DH5alfa.

The processes for specifically designing and preparing the present DNA oligonucleotides as summarised hereinafter. For detailed information, reference is made to the Examples. In the particular processes, molecular biological techniques, which as such are known from the reference manual by Sambrook J., Fritsch E.F., and Maniatis T., "Molecular Cloning, Book 1,2,3. A Laboratory Manual" (1989), Cold Spring Harbor Laboratory Press, were used in general. For the techniques which are generally used for carrying out the so-called polymerase chain reaction or PCR reaction, reference is made to Arnheim N. and Erlich H., Annual Reviews in Biochemistry (1992) 61: 131-156. DNA is isolated from isolates of root-knot nematodes obtained from vegetable samples, cultures or soil samples. A first PCR reaction is carried out with the DNA from one Meloidogyne species. Short decameric DNA oligonucleotides, which can be used as forward primers as well as as reverse primers, are used in said reaction. When, following a gel electrophoretic separation of the products of this first PCR reaction, a band pattern of DNA fragments (i.e. RAPD fragments, where RAPD = Random Amplified Polymorphic DNA) is obtained, bands which do not occur with other species, and therefore might be species-specific, are looked for. After cloning in a suitable vector, the DNA sequence of a possibly species-specific RAPD fragment is determined. Based on the obtained DNA sequence, DNA oligonucleotides are then designed and synthesised, which can separately be used as forward or as reverse primers in PCR reaction. The sequence of these primers is designed on the basis of the sequence for the possibly species-specific RAPD fragment starting with the decameric RAPD primer sequence or within 100 base pairs thereof. For the "nested" PCR reaction, which is explained below, a sequence is chosen which is present farther within the sequence of the possibly species-specific RAPD fragment. During the design stage, sequences having a stable 5'-end and a slightly less stable 3'-end are looked for, as primers obtained on the basis thereof show less incorrect attachment. The designed primers are longer than the RAPD primers and have a length of, for example, 10-30 nucleotides. Using these synthesised primers in a second PCR reaction, DNA fragments are obtained which are termed SCAR fragments (SCAR = Sequence Characterised Amplified Regions); the primers themselves are termed SCAR primers. The longer the DNA oligonucleotides used as SCAR primers, the more specific they hybridise to a full (or partial) SCAR fragment which is possibly species-specific. Finally, the DNA oligonucleotides used as SCAR primers are analysed for species-specificity, reliability and sensitivity. In this manner, the 20 DNA oligonucleotides according to the present invention were obtained.

In principle, oligonucleotides can be used in different ways for a species-specific detection and identification (using, for example, RFLP analysis with DNA from constitutive genes or with mitochondrial DNA). The present invention relates to a process for determining the presence of a Meloidogyne species, according to which DNA is isolated from a sample to be analysed, and is subsequently subjected to a PCR reaction using a specific DNA oligonucleotide primer, whereupon the presence of a Meloidogyne species is determined on the basis of the DNA products formed by said PCR reaction. Such process is also known from Williamson e.a. (1997), in which the specific detection of the amount of one nematode (i.e. from species M. chitwoodi) is described.

The process according to the present invention is characterised in that a DNA oligonucleotide having a sequence chosen from (a) a DNA sequence chosen from the group consisting of SEQ ID No. 1 to SEQ ID No. 20, or (b) a DNA sequence which under conditions of stringency hybridises with a sequence according to (a) and which is specific for Meloidogyne species, is used as a primer.

Using the process according to the present invention, it is not only possible to specifically detect the presence of DNA of one nematode, but in some cases even of just 1 x 10⁻³ nematode. Furthermore, if a so-called "nested PCR" reaction is carried out using certain DNA oligonucleotides according to the present invention, as explained hereinafter in the Examples, even a sensitivity of detection of 1 x 10⁻⁵ nematode can be achieved. It is clear that such a sensitive detection will enable a timely intervention.

The present invention also relates to a process for simultaneously detecting M. fallax, M. hapla and M. chitwoodi, according to which a combination of the oligonucleotide with SEQ ID No. 5 and No. 2, SEQ ID No. 6 and No. 7 and SEQ ID No. 14 and No. 11 are used as a primer. In this particular PCR reaction, certain conditions, as described in the Examples, are used.

As explained above, certain DNA oligonucleotides according to the present invention are particularly suitable for the specific detection and identification of separate Meloidogyne species. The present invention also relates to a process for separately determining the presence of M. fallax, M. hapla, M. chitwoodi, M. arenaria, M. incognita and M. javanica, according to which the above-mentioned DNA oligonucleotides, having a sequence respectively chosen from SEQ ID No. 1 and SEQ ID No. 2, SEQ ID No. 6 and SEQ ID No. 7, SEQ ID No. 10 and SEQ ID No. 11, SEQ ID No. 15 and SEQ ID No. 16, SEQ ID No. 17 and SEQ ID No. 18, SEQ ID No. 19 and SEQ ID No. 20, are used as primers. The invention also relates to processes according to which the sensitivity of the detection of M. fallax, M. hapla and M. chitwoodi can still be increased, by using for each species the DNA oligonucleotide having a sequence respectively chosen from SEQ ID No. 3 and SEQ ID No. 4, SEQ ID No. 8 and SEQ ID No. 9, and SEQ ID No. 12 and SEQ ID No. 13, in a follow-up PCR reaction.

Finally, the present invention relates to a composition or kit for determining the presence of a Meloidogyne species, containing at least one DNA oligonucleotide according to the present invention, a vector containing such a DNA oligonucleotide or a host cell containing such a vector. Such a kit enables a routine, but still specific, sensitive and reliable detection and identification. Apart from said DNA oligonucleotide, a kit can also contain reagents with which a species-specific determination can be carried out. Furthermore, the kit can contain a DNA sample of the species to be determined, with which control reactions can be carried out. If the kit is meant to show the presence of, for example, three Meloidogyne species in one assay, it is preferable to add a DNA control sample consisting of a mixture of equal amounts of DNA of the particular Meloidogyne species. If, using the kit, a species-specific determination has to be carried out using a PCR reaction, one or more of the following substances can be present in the kit: a PCR mixture which is, for example, ten times concentrated (containing nucleotides, a PCR buffer and at least one species-specific DNA oligonucleotide as a primer), the enzyme which is needed for the PCR reaction (Taq DNA polymerase) and sterile water. The ingredients of the PCR mixture and control DNA can exist in a form carried in beads in a set of reaction vials. The DNA oligonucleotides or the combination of DNA oligonucleotides according to the invention is chosen in function of the process for determining the presence of one or more Meloidogyne species using the kit. Obviously, the kit can contain instructions for use.

The invention is further illustrated with examples of preparations, embodiments and uses of the DNA oligonucleotides. In the added sequence listing, the individual sequences are described in the conventional 5'→3' direction. Furthermore, it is indicated for each sequence whether the particular DNA oligonucleotides can be used as a forward primer or as a reverse primer.

### Example 1.

In the present example, a process for preparation DNA from Meloidogyne nematodes and for obtaining possibly species-specific RAPD fragments is described.

Nematodes were collected and concentrated from isolates from the Meloidogyne species M. fallax, M. hapla, M. chitwoodi, M. arenaria, M. incognita and M. javanica using a centrifugation of 2 minutes at 2000 g in a 30% sucrose solution, followed by washing in distilled water. For the extraction, nematodes were pellatised in a microcentrifuge. Pellet material was frozen in liquid nitrogen, treated with protein K and extracted with phenol. The DNA of the water phase was precipitated by adding ethanol. Before use, the DNA was centrifuged and dissolved in a suitable buffer.

DNA from different Meloidogyne species was subjected to a first PCR reaction in a mixture with a volume of 50 µl, having the following composition: 10 mM Tris, pH 9.0, 1.5 mM MgCl₂, 50 mM KCl, 200 µM of each of dATP, dCTP, dGTP and dTTP, 0.3 µM decameric RAPD primer, one unit of Taq DNA polymerase (Pharmacia, Sweden), an amount of 3 ng of total DNA. The used decameric primers were synthesised by Operon Technologies (Alameda, CA, USA). For the amplifications of the first PCR reaction (the RAPD amplifications), the PCR instrument, the so-called thermocycler, was programmed for 45 cycles of 1 minute at 94 °C, 2 minutes at 38 °C and 3 minutes at 72 °C each, with a temperature ramp of 1 °C per 5 seconds for the transition of 38 °C to 72 °C. The composition of the PCR products was analysed using gel electrophoresis in a gel containing 1% agarose, 0.5 x TBE buffer (0.045 M Tris-borate and 0.001 M EDTA) and 0.5 µg/ml ethidium bromide. The voltage was 1-5 V/cm gel. The obtained DNA pattern was visualised by illuminating the gel with ultraviolet light. In order to avoid misinterpretations of the obtained patterns of RAPD fragments, control reactions without template DNA were carried out.

At first, 30 decameric primers were screened on six DNA samples consisting of combined DNA from isolates of the species M. hapla, M. chitwoodi, M. fallax, M. incognita, M. arenaria and M. javanica, in order to determine which of these primers yielded strong amplifications. Most primers yielded good amplification products and were then studied in PCR reactions using DNA of separate Meloidogyne isolates. The obtained RAPD band patterns were studied for the presence of bands not occurring in the pattern of any other species, and which therefore possibly contained species-specific RAPD fragments. For each of said six Meloidogyne species, at least one RAPD fragment was found that was truly species-specific, as is shown in the following example. The species-specific RAPD fragments used in the following examples, also termed RAPD markers, are: OPI-01₅₆₀ for M. fallax, PI-14₆₁₀ for M. hapla, OPI-09₉₀₀ for M. chitwoodi, OPA-12₄₂₀ for M. arenaria, OPB-06₁₂₀₀ for M. incognita and OPA-01₇₀₀ for M. javanica. In the abbreviations, the subindex refers to the length of the particular fragment, expressed in base pairs.

### Example 2.

In the present example, a process for designing and preparing species-specific DNA oligonucleotides, as well as the species-specific determination of Meloidogyne species, are described.

Except for OPB-06₁₂₀₀, the species-specific RAPD fragments obtained in Example 1 were purified from the gel and were cloned in vector pGEM-T (Promega), using the instructions of the manufacturer. DNA from OPB-06₁₂₀₀ was cloned in vector pCR2.1-TOPO, using the TOPO TA Cloning Kit from Invitrogen. The cloned DNA inserts were tested using amplification of so-called miniprep-plasmid DNA with the original RAPD primers. In summary, said process comprises the following. An overnight culture of 2 ml was centrifuged in a table centrifuge during 15 seconds. The supernatant was discarded and an amount of 200 µl lysozyme solution (2.5 M LiCl, 0.05 M Tris, pH 7.5, 0.06 M EDTA, 0.4% Triton, 1 mg/ml lysozyme) was added to the bacteria. Following thorough mixing, the bacteria were cooked during 1.5 minutes, kept on ice during 5 minutes and were finally centrifuged at full speed in a table centrifuge during 18 minutes. The obtained pellets were discarded, and the DNA in solution was precipitated with ethanol. Following centrifugation, the obtained DNA pellet was dried and dissolved in 20 µl water. An amount of 3 µl thereof was used for a PCR reaction with the original RAPD primer. If in this PCR reaction a DNA fragment with the expected size was obtained, it was concluded that the particular plasmid contained the desired RAPD fragment.

Plasmid DNA from clones with a correct insert was extracted using a plasmid kit from Qiagen (Hilden, Germany), following the instructions of the manufacturer. The inserts were sequenced in both directions, using a process based on the method according to Sanger.

Based on the sequence of fragment OPI-01₅₆₀, which is specific for M. fallax, forward and reverse primers were designed (these are so-called SCAR primers, as explained above). From the synthesised primers, two such primers were chosen which, in a PCR amplification, yielded the clearest and most species-specific signal, i.e. gel electrophoretic band of the PCR amplification product. The sequences of the particular primers are mentioned as SEQ ID No. 1 and SEQ ID No. 2 in the sequence listing. The DNA oligonucleotides were synthesised in a conventional way, using an automatic DNA synthesizer.

The PCR amplification reactions for the particular SCAR fragment were carried out in a reaction volume of 50 µl, containing 10 mM Tris, pH 9.0, 1.5 mM MgCl₂, 50 mM KCl, 200 µM each of dATP, dCTP, dGTP and dTTP, 1 unit of Taq DNA polymerase (Pharmacia), an amount of 3 ng of total DNA from M. fallax to be analysed. For such a "normal" PCR reaction using two primers, in this case having sequences SEQ ID No. 1 and SEQ ID No. 2, they were used at final concentrations of 0.3 µM. For the PCR amplification reactions of the SCAR fragment, the thermocycler of the PCR instrument was programmed for 2 minutes at 94 °C, followed by 30 cycles of 30 seconds at 94 °C, 30 seconds at the particular annealing temperature of 58 °C, and 1 minute at 72 °C each. The composition of the obtained PCR products was analysed as described in Example 1. The amplification of the fragment with a length of 515 base pairs (bp) was shown to be species-specific. When DNA from M. hapla, M. chitwoodi, M. incognita, M. arenaria or M. javanica was used, no amplification could be observed. Therefore, the aforementioned primers are species-specific for M. fallax.

In a similar way, forward and reverse primers were designed on the basis of the sequence of fragment OPI-14₆₁₀, which is specific for M. hapla. The sequences of the chosen primers are mentioned as SEQ ID No. 6 and SEQ ID No. 7 in the sequence listing. During SCAR-PCR amplification of DNA from M. hapla, using an annealing temperature of 60 °C, amplification of a fragment with a length of 610 bp was obtained. When DNA from M. fallax, M. chitwoodi, M. incognita, M. arenaria or M. javanica was used, no amplification could be observed. Therefore, the SCAR primers with the aforementioned sequences are species-specific for M. hapla.

In a similar way, forward and reverse primers were designed on the basis of the sequence of fragment OPI-09₉₀₀, which is specific for M. chitwoodi. The sequences of the chosen primers are mentioned as SEQ ID No. 10 and SEQ ID No. 11 in the sequence listing. During SCAR-PCR amplification of DNA from M. chitwoodi, using an annealing temperature of 60 °C, amplification of a fragment with a length of 800 bp was obtained. When DNA from M. fallax, M. hapla, M. incognita, M. arenaria or M. javanica was used, no amplification could be observed. Therefore, the SCAR primers with the aforementioned sequences are species-specific for M. chitwoodi.

In a similar way, forward and reverse primers were designed on the basis of the sequence of fragment OPA-12₄₂₀, which is specific for M. arenaria. The sequences of the chosen primers are mentioned as SEQ ID No. 15 and SEQ ID No. 16 in the sequence listing. During SCAR-PCR amplification of DNA from M. arenaria, using an annealing temperature of 61 °C, amplification of a fragment with a length of 420 bp was obtained. When DNA from M. fallax, M. hapla, M. chitwoodi, M. incognita or M. javanica was used, no amplification could be observed. Therefore, the SCAR primers with the aforementioned sequences are species-specific for M. arenaria.

In a similar way, forward and reverse primers were designed on the basis of the sequence of fragment OPB-06₁₂₀₀, which is specific for M. incognita. The sequences of the chosen primers are mentioned as SEQ ID No. 17 and SEQ ID No. 18 in the sequence listing. During SCAR-PCR amplification of DNA from M. incognita, using an annealing temperature of 54 °C, amplification of a fragment with a length of 1200 bp was obtained. When DNA from M. fallax, M. hapla, M. chitwoodi, M. arenaria or M. javanica was used, no amplification could be observed. Therefore, the SCAR primers with the aforementioned sequences are species-specific for M. incognita.

In a similar way, forward and reverse primers were designed on the basis of the sequence of fragment OPA-01₇₀₀, which is specific for M. javanica. The sequences of the chosen primers are mentioned as SEQ ID No. 19 and SEQ ID No. 20 in the sequence listing. During SCAR-PCR amplification of DNA from M. javanica, using an annealing temperature of 64 °C, amplification of a fragment with a length of 670 bp was obtained. When DNA from M. fallax, M. hapla, M. chitwoodi, M. incognita or M. arenaria was used, no amplification could be observed. Therefore, the SCAR primers with the aforementioned sequences are species-specific for M. javanica.

### Example 3.

The present example describes the process for designing and preparing DNA oligonucleotides for the simultaneous and species-specific detection of M. fallax, M. hapla and M. chitwoodi.

Using the processes as described in Examples 1 and 2, SCAR primers were designed and synthesised to demonstrate the presence of DNA from the above-mentioned three Meloidogyne species simultaneously in one PCR reaction. The chosen SCAR primers had the sequences which are mentioned in the sequence listing as SEQ ID No. 5 and SEQ ID No. 2 for M. fallax, the sequences SEQ ID No. 6 and SEQ ID No. 7 for M. hapla, and the sequences SEQ ID No. 14 and SEQ ID No. 11 for M. chitwoodi, respectively.

In order to optimise the simultaneous detection of the above-mentioned Meloidogyne species in one so-called multiplex PCR reaction (in such a way that the intensities of the three SCAR-DNA fragments were comparable when using the 1:1:1 mixture of DNA from M. fallax, M. hapla and M. chitwoodi), the reaction conditions for the SCAR-PCR amplification reaction as described in Example 2 were changed: the primer mixture consisted of 0.18 µM for the SCAR primers with the respective sequences SEQ ID No. 5 and SEQ ID No. 2, 0.3 µM for the SCAR primers with sequences SEQ ID No. 6 and SEQ ID No. 7, and 0.15 µM for the SCAR primers with sequences SEQ ID No. 14 and SEQ ID No. 11. The annealing temperature of the PCR program was 58 °C. The number of the cycles was 43.

The obtained PCR reaction products were analysed as described in Example 1. The results of this multiplex PCR reaction show that, on the basis of the obtained SCAR fragments with a length of 530 bp, 610 bp and 755 bp, respectively, the species M. fallax, M. hapla and M. chitwoodi could be easily distinguished from each other, even if the DNA of the species is present in a mixture.

When the mutual proportion of the DNA from the three Meloidogyne species was changed, it was shown that a relative amount of 2% to 5% of M. Chitwoodi, M. hapla or M. fallax could be detected.

### Example 4.

In the present example, the design and preparation of species-specific DNA oligonucleotides in the form of so-called "nested" primers, as well as a species-specific detection with increased sensitivity of Meloidogyne species are described.

For the species M. fallax, M. hapla and M. chitwoodi, "nested" primers were developed on the basis of a sequence of the respective RAPD markers OPI-01₅₆₀, OPI-14₆₁₀ and OPI-09₉₀₀, and lying more than 100 base pairs (bp) from the end of the markers. From the different synthesised DNA oligonucleotides, those which as primers in a "nested" PCR reaction, which was carried out with species-specific SCAR-PCR product obtained by using a normal PCR reaction (as described in Example 2), yielded the clearest and most species-specific amplification products (in this particular case nested PCR products), were chosen. As described in Example 5, the sensitivity of the detection of the particular Meloidogyne species could be increased considerably by carrying out a nested PCR reaction following the normal PCR reaction.

Concerning M. fallax, the DNA oligonucleotides having sequences SEQ ID No. 3 and SEQ ID No. 4 were chosen as primers for a nested PCR reaction with the SCAR product obtained when using a "normal" PCR reaction using primers having sequences SEQ ID No. 1 and SEQ ID No. 2 (see Example 2).

Concerning M. hapla, the DNA oligonucleotides having sequences SEQ ID No. 8 and SEQ ID No. 9 were chosen as primers for a nested PCR reaction with the SCAR product obtained when using a "normal" PCR reaction using primers having sequences SEQ ID No. 6 and SEQ ID No. 7 (see Example 2).

Concerning M. chitwoodi, the DNA oligonucleotides having sequences SEQ ID No. 12 and SEQ ID No. 13 were chosen as primers for a nested PCR reaction with the SCAR product obtained when using a "normal" PCR reaction using primers having sequences SEQ ID No. 10 and SEQ ID No. 11 (see Example 2).

The nested PCR reactions were carried out in a reaction volume of 50 µl containing 10 mM Tris, pH 9.0, 1.5 mM MgCl₂, 50 mM KCl, 200 µM each of dATP, dCTP, dGTP and dTTP, 1 unit of Taq DNA polymerase (Pharmacia), 2 µl of one of the above-mentioned SCAR-PCR products and one of the above-mentioned sets of primers with a final concentration of 0.3 µM. For the nested PCR reaction, the thermocycler of the PCR instrument was programmed for 2 minutes at 94 °C, followed by 30 cycles of 30 seconds at 94 °C, 30 seconds at the annealing temperature (64 °C for the above-mentioned combination which is specific for M. fallax; 60 °C for the above-mentioned combination which is specific for M. hapla; 59 °C for the above-mentioned combination which is specific for M. chitwoodi), and 1 minute at 72 °C. The composition of the obtained PCR products was analysed as described in Example 1. It was shown that each of the above-mentioned combinations can be used for a species-specific detection of the particular Meloidogyne species. The increased sensitivity of detection compared with a species-specific determination based on a normal SCAR-PRC reaction as described in Example 2, is described in the following Example 5.

### Example 5.

In the present example, the sensitivity of the detection of the processes according to the present invention is illustrated. DNA from different Meloidogyne species was prepared as described in Example 1. A crude extract from one single second stage juvenile form of the Meloidogyne species to be studied was obtained by using the method according to Harris, T.S., Sandall, L.J. and Powers, T.O. (1990), Journal of Nematology, 22, 518-524.

A normal SCAR-PCR reaction was carried out as described in Example 2, except for the DNA used as a template, which consisted of very small amounts of the target DNA of a particular Meloidogyne species, as indicated below, mixed with an amount of 10 ng of DNA from a different species.

The results showed that an amount of DNA corresponding to 0.1% of the amount of DNA extracted from one single juvenile form of M. hapla was sufficient for the species-specific amplification of the SCAR DNA fragment with a length of 610 bp, using the primers having sequences SEQ ID No. 6 and SEQ ID No. 7. Using this normal SCAR-PCR reaction, it was shown that an amount of DNA corresponding to 0.01% of the DNA of one single juvenile form of M. hapla was no longer detectable. The detection limit for DNA of M. fallax and M. chitwoodi was determined in a similar way.

The detection limit obtained when using a normal SCAR-PCR reaction could be increased by a factor 10⁴ to 10⁵ by using the respective SCAR-DNA products as template DNA for a subsequent nested PCR reaction (as described in Example 4). For M. fallax, the PCR product of a normal SCAR-PCR reaction (a DNA fragment with a length of 515 bp) using primers having sequences SEQ ID No. 1 and SEQ ID No. 2, and an amount of DNA corresponding to 10⁻⁵ juvenile form of M. fallax, was used as template DNA for a subsequent nested PCR reaction using primers having sequences SEQ ID No. 3 and SEQ ID No. 4. It was shown that the obtained nested PCR product with a length of 200 bp could still be detected when using the gel electrophoresis as described in Example 1.

### Example 6.

The present example relates to a kit for the specific determination of the presence of Meloidogyne fallax, using a PCR reaction.

A kit for 100 assays, which had the following composition, was assembled: a container with a tenfold concentrated PCR mixture (consisting of dATP, dCTP, dGTP, dTTP, Tris as PCR buffer, the DNA oligonucleotide having sequence SEQ ID No. 1 and the DNA oligonucleotide having sequence SEQ ID No. 2), a container with Taq DNA polymerase (100 µl, 1 U/µl, in 50 mM Tris-HCl, pH 8.0, 1 mM EDTA, 1 mM DTT, 50% (g/g) glycerol, stabilising agents), a container with 300 ng of DNA from Meloidogyne fallax (1 ng/µl in 10 mM Tris-HCl, pH 7.5, 1 mM EDTA), and a container with sterile water. Finally, instructions for use, containing precise instructions and guidelines for carrying out the PCR reaction and the control PCR reaction, as well as an advise concerning the interpretation of the results, were added.

### Example 7.

The present example relates to a kit for the simultaneous and specific determination of the presence of Meloidogyne fallax, M. hapla and M. chitwoodi, using a PCR reaction.

A kit for 100 assays, which had a composition analogous to that of the kit of Example 6, except for the DNA oligonucleotides and the control DNA, was assembled. The species-specific DNA oligonucleotides were oligonucleotides having the respective sequences SEQ ID No. 5, SEQ ID No. 2, SEQ ID No. 6, SEQ ID No. 7, SEQ ID No. 14 and SEQ ID No. 11. The control DNA consisted of a 1:1:1 mixture of DNA from M. fallax, M. hapla and M. chitwoodi (an amount of 100 ng of each species, 1 ng/µl in 10 mM Tris-HCl, pH 7.5, 1 mM EDTA.

### SEQUENCE LISTING

### GENERAL INFORMATION:

APPLICANT:
   NAME:Stichting Dienst Landbouwkundig Onderzoek (IPO-DLO)
   STREET:Bornsesteeg 53
   CITY:WAGENINGEN
   COUNTRY:The Netherlands
   POSTAL CODE:6708 PD
TITLE OF INVENTION: DNA oligonucleotide which is specific for Meloidogyne species, DNA vector and host cell containing said oligonucleotide, use and kit.
NUMBER OF SEQUENCES: 20
COMPUTER READABLE FORM:
   MEDIUM TYPE COMPUTER: HD-diskette
   COMPUTER: IBM-compatible PC
   OPERATING SYSTEM: DOS
   SOFTWARE:WP 5.1
PRIOR APPLICATION DATA:
   APPLICATION NUMBER: NL 1010917
   FILING DATE: December 30, 1998
ATTORNEY / AGENT INFORMATION:
   NAME:
      Algemeen Octrooibureau
      Ir. J.J.H. Van kan c.s.
      P.O. Box 645, 5600 AP EINDHOVEN, The Netherlands
      TELEPHONE:040-2433715
      TELEFAX:040-2434557

### INFORMATION FOR SEQ ID No. 1

SEQUENCE CHARACTERISTICS:
   LENGTH: 23 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 1:

CCAAACTATC GTAATGCATT ATT 23

### INFORMATION FOR SEQ ID No. 2

SEQUENCE CHARACTERISTICS:
   LENGTH: 23 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 2:

GGACACAGTA ATTCATGAGC TAG 23

### INFORMATION FOR SEQ ID No. 3

SEQUENCE CHARACTERISTICS:
   LENGTH: 20 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 3:

GCACCGAAAC GTCCTGTAAA 20

### INFORMATION FOR SEQ ID No. 4

SEQUENCE CHARACTERISTICS:
   LENGTH: 22 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 4:

CTGCGTTGAA CCGACCTATA CT 22

### INFORMATION FOR SEQ ID No. 5

SEQUENCE CHARACTERISTICS:
   LENGTH: 24 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 5:

CCATTTCTGC TAAATGCCAA ACTA 24

### INFORMATION FOR SEQ ID No. 6

SEQUENCE CHARACTERISTICS:
   LENGTH: 18 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 6:

TGACGGCGGT GAGTGCGA 18

### INFORMATION FOR SEQ ID No. 7

SEQUENCE CHARACTERISTICS:
   LENGTH: 19 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 7:

TGACGGCGGT ACCTCATAG 19

### INFORMATION FOR SEQ ID No. 8

SEQUENCE CHARACTERISTICS:
   LENGTH: 21 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 8:

GCCTTCTTTG GATTCCTCTC A 21

### INFORMATION FOR SEQ ID No. 9

SEQUENCE CHARACTERISTICS:
   LENGTH: 20 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 9:

GGCTCATCCT TGCTGTAAAT 20

### INFORMATION FOR SEQ ID No. 10

SEQUENCE CHARACTERISTICS:
   LENGTH: 21 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 10:

TGGAGAGCAG CAGGAGAAAG A 21

### INFORMATION FOR SEQ ID No. 11

SEQUENCE CHARACTERISTICS:
   LENGTH: 22 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 11:

GGTCTGAGTG AGGACAAGAG TA 22

### INFORMATION FOR SEQ ID No. 12

SEQUENCE CHARACTERISTICS:
   LENGTH: 20 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 12:

CGCTGATAAT CAGAGCAAAC 20

### INFORMATION FOR SEQ ID No. 13

SEQUENCE CHARACTERISTICS:
   LENGTH: 23 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 13:

GCCAATTCAT AAGTGTTGTC TAG 23

### INFORMATION FOR SEQ ID No. 14

SEQUENCE CHARACTERISTICS:
   LENGTH: 22 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 14:

GGCATTGACG TGCTCCGAGA GT 22

### INFORMATION FOR SEQ ID No. 15

SEQUENCE CHARACTERISTICS:
   LENGTH: 21 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 15:

TCGGCGATAG AGGTAAATGA C 21

### INFORMATION FOR SEQ ID No. 16

SEQUENCE CHARACTERISTICS:
   LENGTH: 21 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 16:

TCGGCGATAG ACACTACAAC T 21

### INFORMATION FOR SEQ ID No. 17

SEQUENCE CHARACTERISTICS:
   LENGTH: 20 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 17:

CTCTGCCCAA TGAGCTGTCC 20

### INFORMATION FOR SEQ ID No. 18

SEQUENCE CHARACTERISTICS:
   LENGTH: 18 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 18:

CTCTGCCCTC ACATTAAG 18

### INFORMATION FOR SEQ ID No. 19

SEQUENCE CHARACTERISTICS:
   LENGTH: 20 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: forward primer
SEQUENCE DESCRIPTION: SEQ ID No. 19:

GGTGCGCGAT TGAACTGAGC 20

### INFORMATION FOR SEQ ID No. 20

SEQUENCE CHARACTERISTICS:
   LENGTH: 23 bases
   TYPE: Nucleic acid
   STRANDEDNESS: single
   TOPOLOGY: linear
MOLECULE TYPE: Synthetic DNA oligonucleotide
FEATURE:
   OTHER INFORMATION: reverse primer
SEQUENCE DESCRIPTION: SEQ ID No. 20:

CAGGCCCTTC AGTGGAACTA TAC 23

## Claims

1. A DNA oligonucleotide which is specific for Meloidogyne species, **characterised in that** the DNA oligonucleotide has the following sequence:
(a) a DNA sequence chosen from the group consisting of SEQ ID No. 1 to SEQ ID No. 20, or
(b) a DNA sequence which under conditions of stringency hybridises with a sequence according to (a) and which is specific for Meloidogyne species.

2. A DNA oligonucleotide according to claim 1, **characterised in that** it consists of a combination of the oligonucleotide with SEQ ID No. 5 and No. 2, SEQ ID No. 6 and No. 7 and SEQ ID No. 14 and No. 11.

3. A DNA oligonucleotide according to claim 1, **characterised in that** it is an oligonucleotide having a sequence chosen from SEQ ID No. 1 and SEQ ID No. 2.

4. A DNA oligonucleotide according to claim 1, **characterised in that** it is an oligonucleotide having a sequence chosen from SEQ ID No. 6 and SEQ ID No. 7.

5. A DNA oligonucleotide according to claim 1, **characterised in that** it is an oligonucleotide having a sequence chosen from SEQ ID No. 10 and SEQ ID No. 11.

6. A DNA oligonucleotide according to claim 1, **characterised in that** it is an oligonucleotide having a sequence chosen from SEQ ID No. 15 and SEQ ID No. 16.

7. A DNA oligonucleotide according to claim 1, **characterised in that** it is an oligonucleotide having a sequence chosen from SEQ ID No. 17 and SEQ ID No. 18.

8. A DNA oligonucleotide according to claim 1, **characterised in that** it is an oligonucleotide having a sequence chosen from SEQ ID No. 19 and SEQ ID No. 20.

9. A DNA oligonucleotide according to claim 1, **characterised in that** it is an oligonucleotide having a sequence chosen from SEQ ID No. 3 and SEQ ID No. 4.

10. A DNA oligonucleotide according to claim 1, **characterised in that** it is an oligonucleotide having a sequence chosen from SEQ ID No. 8 and SEQ ID No. 9.

11. A DNA oligonucleotide according to claim 1, **characterised in that** it is an oligonucleotide having a sequence chosen from SEQ ID No. 12 and SEQ ID No. 13.

12. A DNA vector containing a DNA oligonucleotide according to any one of claims 1-11.

13. Host cell containing vector according to claim 12.

14. A process for determining the presence of a Meloidogyne species, according to which DNA is isolated from a sample to be analysed, and is subsequently subjected to a PCR reaction using a specific DNA oligonucleotide primer, whereupon the presence of a Meloidogyne species is determined on the basis of the DNA products formed by said PCR reaction, **characterised in that** a DNA oligonucleotide having a sequence chosen from
(a) a DNA sequence chosen from the group consisting of SEQ ID No. 1 to SEQ ID No. 20, or
(b) a DNA sequence which under conditions of stringency hybridises with a sequence according to (a) and which is specific for Meloidogyne species,
is used as a primer.

15. A process according to claim 14, **characterised in that** the presence of M. fallax, M. hapla, and M. chitwoodi is determined simultaneously by using the DNA oligonucleotide according to claim 2 as a primer.

16. A process according to claim 14, **characterised in that** the presence of M. fallax is determined by using the DNA oligonucleotide according to claim 3 as primer.

17. A process according to claim 14, **characterised in that** the presence of M. hapla is determined by using the DNA oligonucleotide according to claim 4 as primer.

18. A process according to claim 14, **characterised in that** the presence of M. chitwoodi is determined by using the DNA oligonucleotide according to claim 5 as primer.

19. A process according to claim 14, **characterised in that** the presence of M. arenaria is determined by using the DNA oligonucleotide according to claim 6 as primer.

20. A process according to claim 14, **characterised in that** the presence of M. incognita is determined by using the DNA oligonucleotide according to claim 7 as primer.

21. A process according to claim 14, **characterised in that** the presence of M. javanica is determined by using the DNA oligonucleotide according to claim 8 as primer.

22. A process according to claim 16, **characterised in that** the DNA oligonucleotide according to claim 9 is used as a primer in a follow-up PCR reaction.

23. A process according to claim 17, **characterised in that** the DNA oligonucleotide according to claim 10 is used as a primer in a follow-up PCR reaction.

24. A process according to claim 18, **characterised in that** the DNA oligonucleotide according to claim 11 is used as a primer in a follow-up PCR reaction.

25. Kit for determining the presence of a Meloidogyne species, containing at least one DNA oligonucleotide according to any one of claims 1-11, a vector according to claim 12 or a host cell according to claim 13.

## Patentansprüche

1. DNA-Oligonukleotid, welches spezifisch für Meloidogyne-Spezies ist, **dadurch gekennzeichnet, dass** das DNA-Oligonuklotid die folgende Sequenz besitzt:
(a) DNA-Sequenz ausgewählt aus SEQ ID Nr. 1 bis SEQ ID Nr. 20, oder
(b) DNA-Sequenz, welche unter zwingenden Bedingungen mit einer Sequenz gemäß (a) hybridisiert, und welche spezifisch für Meloidogyne-Spezies ist.

2. DNA-Oligonukleotid nach Anspruch 1, **dadurch gekennzeichnet, dass** es aus einer Kombination des Oligonuklotids mit SEQ ID Nr. 5 und Nr. 2, SEQ ID Nr. 6 und Nr. 7 und SEQ ID Nr. 14 und Nr. 11 besteht.

3. DNA-Oligonukleotid nach Anspruch 1, **dadurch gekennzeichnet, dass** es ein Oligonukleotid mit einer Sequenz ist, die aus SEQ ID Nr. 1 und SEQ ID Nr. 2 ausgewählt ist.

4. DNA-Oligonukleotid nach Anspruch 1, **dadurch gekennzeichnet, dass** es ein Oligonukleotid mit einer Sequenz ist, die aus SEQ ID Nr. 6 und SEQ ID Nr. 7 ausgewählt ist.

5. DNA-Oligonukleotid nach Anspruch 1, **dadurch gekennzeichnet, dass** es ein Oligonukleotid mit einer Sequenz ist, die aus SEQ ID Nr. 10 und SEQ ID Nr. 11 ausgewählt ist.

6. DNA-Oligonukleotid nach Anspruch 1, **dadurch gekennzeichnet, dass** es ein Oligonukleotid mit einer Sequenz ist, die aus SEQ ID Nr. 15 und SEQ ID Nr. 16 ausgewählt ist.

7. DNA-Oligonukleotid nach Anspruch 1, **dadurch gekennzeichnet, dass** es ein Oligonukleotid mit einer Sequenz ist, die aus SEQ ID Nr. 17 und SEQ ID Nr. 18 ausgewählt ist.

8. DNA-Oligonukleotid nach Anspruch 1, **dadurch gekennzeichnet, dass** es ein Oligonukleotid mit einer Sequenz ist, die aus SEQ ID Nr. 19 und SEQ ID Nr. 20 ausgewählt ist.

9. DNA-Oligonukleotid nach Anspruch 1, **dadurch gekennzeichnet, dass** es ein Oligonukleotid mit einer Sequenz ist, die aus SEQ ID Nr. 3 und SEQ ID Nr. 4 ausgewählt ist.

10. DNA-Oligonukleotid nach Anspruch 1, **dadurch gekennzeichnet, dass** es ein Oligonukleotid mit einer Sequenz ist, die aus SEQ ID Nr. 8 und SEQ ID Nr. 9 ausgewählt ist.

11. DNA-Oligonukleotid nach Anspruch 1, **dadurch gekennzeichnet, dass** es ein Oligonukleotid mit einer Sequenz ist, die aus SEQ ID Nr. 12 und SEQ ID Nr. 13 ausgewählt ist.

12. DNA-Vektor, der ein DNA-Oligonukleotid nach einem der Ansprüche 1-11 enthält.

13. Wirtszelle, die einen Vektor nach Anspruch 12 enthält.

14. Verfahren zur Bestimmung der Anwesenheit einer Meloidogyne-Spezies, nach dem DNA aus einer zu analysierenden Probe isoliert wird und anschließend einer PCR-Reaktion mit einem spezifischen DNA-Oligonukleotid-Primer unterzogen wird, woraufhin die Anwesenheit von Meloidogyne-Spezies auf Basis der durch die genannte PCR-Reaktion gebildeten DNA-Produkte bestimmt wird, **dadurch gekennzeichnet, dass** ein DNA-Oligonukleotid mit einer Sequenz ausgewählt aus
(a) einer DNA-Sequenz ausgewählt aus SEQ ID Nr. 1 bis SEQ ID Nr. 20, oder
(b) einer DNA-Sequenz, welche unter zwingenden Bedingungen mit einer Sequenz gemäß (a) hybridisiert, und welche spezifisch für Meloidogyne-Spezies ist,
als Primer verwendet wird.

15. Verfahren nach Anspruch 14, **dadurch gekennzeichnet, dass** die Anwesenheit von M. fallax, M. hapla und M. chitwoodi gleichzeitig bestimmt wird durch die Verwendung des DNA-Oligonukleotids nach Anspruch 2 als Primer.

16. Verfahren nach Anspruch 14, **dadurch gekennzeichnet, dass** die Anwesenheit von M. fallax bestimmt wird durch die Verwendung des DNA-Oligonukleotids nach Anspruch 3 als Primer.

17. Verfahren nach Anspruch 14, **dadurch gekennzeichnet, dass** die Anwesenheit von M. hapla bestimmt wird durch die Verwendung des DNA-Oligonukleotids nach Anspruch 4 als Primer.

18. Verfahren nach Anspruch 14, **dadurch gekennzeichnet, dass** die Anwesenheit von M. chitwoodi bestimmt wird durch die Verwendung des DNA-Oligonukleotids nach Anspruch 5 als Primer.

19. Verfahren nach Anspruch 14, **dadurch gekennzeichnet, dass** die Anwesenheit von M. arenaria bestimmt wird durch die Verwendung des DNA-Oligonukleotids nach Anspruch 6 als Primer.

20. Verfahren nach Anspruch 14, **dadurch gekennzeichnet, dass** die Anwesenheit von M. incognita bestimmt wird durch die Verwendung des DNA-Oligonukleotids nach Anspruch 7 als Primer.

21. Verfahren nach Anspruch 14, **dadurch gekennzeichnet, dass** die Anwesenheit von M. javanica bestimmt wird durch die Verwendung des DNA-Oligonukleotids nach Anspruch 8 als Primer.

22. Verfahren nach Anspruch 16, **dadurch gekennzeichnet, dass** das DNA-Oligonukleotid nach Anspruch 9 als Primer in einer nachfolgenden PCR-Reaktion benutzt wird.

23. Verfahren nach Anspruch 17, **dadurch gekennzeichnet, dass** das DNA-Oligonukleotid nach Anspruch 10 als Primer in einer nachfolgenden PCR-Reaktion benutzt wird.

24. Verfahren nach Anspruch 18, **dadurch gekennzeichnet, dass** das DNA-Oligonukleotid nach Anspruch 11 als Primer in einer nachfolgenden PCR-Reaktion benutzt wird.

25. Kit zur Bestimmung der Anwesenheit einer Meloidogyne-Spezies, das mindestens ein DNA-Oligonukleotid nach einem der Ansprüche 1-11, einen Vektor nach Anspruch 12 oder eine Wirtszelle nach Anspruch 13 enthält.

## Revendications

1. Oligonucléotide d'ADN qui est spécifique d'espèces du genre Meloidogyne, **caractérisé en ce que** l'oligonucléotide d'ADN a la séquence suivante :
(a) une séquence d'ADN choisie dans le groupe composé de la SEQ ID N°1 à la SEQ. ID. N°20, ou
(b) une séquence d'ADN qui, dans des conditions de stringence, s'hybride avec une séquence selon (a) et qui est spécifique d'espèces du genre Meloidogyne.

2. Oligonucléotide d'ADN selon la revendication 1, **caractérisé en ce qu'**il est constitué d'une combinaison des oligonucléotides ayant la SEQ ID N°5 et N°2, la SEQ. ID. N°6 et N°7 et la SEQ ID N°14 et N°11.

3. Oligonucléotide d'ADN selon la revendication 1, **caractérisé en ce que** c'est un oligonucléotide ayant une séquence choisie parmi la SEQ ID N°1 et la SEQ. ID N°2.

4. Oligonucléotide d'ADN selon la revendication 1, **caractérisé en ce que** c'est un oligonucléotide ayant une séquence choisie parmi la SEQ ID N°6 et la SEQ. ID N°7.

5. Oligonucléotide d'ADN selon la revendication 1, **caractérisé en ce que** c'est un oligonucléotide ayant une séquence choisie parmi la SEQ ID N°10 et la SEQ. ID N°11.

6. Oligonucléotide d'ADN selon la revendication 1, **caractérisé en ce que** c'est un oligonucléotide ayant une séquence choisie parmi la SEQ ID N°15 et la SEQ. ID N°16.

7. Oligonucléotide d'ADN selon la revendication 1, **caractérisé en ce que** c'est un oligonucléotide ayant une séquence choisie parmi la SEQ ID N°17 et la SEQ. ID N°18.

8. Oligonucléotide d'ADN selon la revendication 1, **caractérisé en ce que** c'est un oligonucléotide ayant une séquence choisie parmi la SEQ ID N°19 et la SEQ. ID N°20.

9. Oligonucléotide d'ADN selon la revendication 1, **caractérisé en ce que** c'est un oligonucléotide ayant une séquence choisie parmi la SEQ ID N°3 et la SEQ. ID N°4.

10. Oligonucléotide d'ADN selon la revendication 1, **caractérisé en ce que** c'est un oligonucléotide ayant une séquence choisie parmi la SEQ ID N°8 et la SEQ. ID N°9.

11. Oligonucléotide d'ADN selon la revendication 1, **caractérisé en ce que** c'est un oligonucléotide ayant une séquence choisie parmi la SEQ ID N°12 et la SEQ. ID N°13.

12. Vecteur d'ADN contenant un oligonucléotide d'ADN selon l'une quelconque des revendications 1 à 11.

13. Cellule-hôte contenant un vecteur selon la revendication 12.

14. Procédé de détermination de la présence d'une espèce du genre Meloidogyne, selon lequel l'ADN est isolé à partir d'un échantillon qui doit être analysé, et est ensuite soumis à une réaction de PCR en utilisant une amorce d'oligonucléotide d'ADN spécifique, la présence d'une espèce du genre Meloidogyne étant alors déterminée sur la base des produits ADN formés par ladite réaction de PCR, **caractérisé en ce qu'**un oligonucléotide d'ADN ayant une séquence choisie parmi
(a) une séquence d'ADN choisie dans le groupe composé de la SEQ ID N°1 à SEQ. ID. N°20, ou
(b) une séquence d'ADN qui, dans des conditions de stringence, s'hybride avec une séquence selon (a) et qui est spécifique d'espèces du genre Meloidogyne,
est utilisé à titre d'amorce.

15. Procédé selon la revendication 14, **caractérisé en ce que** la présence de M. fallax, M. hapla, et M. chitwoodi est déterminée simultanément en utilisant l'oligonucléotide d'ADN selon la revendication 2 à titre d'amorce.

16. Procédé selon la revendication 14, **caractérisé en ce que** la présence de M. fallax est déterminée en utilisant l'oligonucléotid d'ADN selon la revendication 3 à titre d'amorce.

17. Procédé selon la revendication 14, **caractérisé en ce que** la présence de M. hapla est déterminée en utilisant l'oligonucléotide d'ADN selon la revendication 4 à titre d'amorce.

18. Procédé selon la revendication 14, **caractérisé en ce que** la présence de M. chitwoodi est déterminée en utilisant l'oligonucléotide d'ADN selon la revendication 5 à titre d'amorce.

19. Procédé selon la revendication 14, **caractérisé en ce que** la présence de M. arenaria est déterminée en utilisant l'oligonucléotide d'ADN selon la revendication 6 à titre d'amorce.

20. Procédé selon la revendication 14, **caractérisé en ce que** la présence de M. incognita est déterminée en utilisant l'oligonucléotide d'ADN selon la revendication 7 à titre d'amorce.

21. Procédé selon la revendication 14, **caractérisé en ce que** la présence de M. javanica est déterminée en utilisant l'oligonucléotide d'ADN selon la revendication 8 à titre d'amorce.

22. Procédé selon la revendication 16, **caractérisé en ce que** l'oligonucléotide d'ADN selon la revendication 9 est utilisé à titre d'amorce dans une réaction de PCR complémentaire.

23. Procédé selon la revendication 17, **caractérisé en ce que** l'oligonucléotide d'ADN selon la revendication 10 est utilisé à titre d'amorce dans une réaction de PCR complémentaire.

24. Procédé selon la revendication 18, **caractérisé en ce que** l'oligonucléotide d'ADN selon la revendication 11 est utilisé à titre d'amorce dans une réaction de PCR complémentaire.

25. Trousse pour la détermination de la présence d'une espèce du genre Meloidogyne contenant au moins un oligonucléotide d'ADN selon l'une quelconque des revendications 1 à 11, un vecteur selon la revendication 12 ou une cellule-hôte selon la revendication 13.
